# EUROPEAN PATENT SPECIFICATION

(11) **EP 2 695 534 B1**
(45) Date of publication and mention of the grant of the patent: **09.11.2016**
(21) Application number: 12197163.4
(22) Date of filing: 14.12.2012
(51) Int. Cl.: A41B 9/00, A41D 7/00

(54) **Clothing such as shorts**
Bekleidung wie etwa kurze Hosen
Vêtements tels que des shorts

(30) Priority: 10.08.2012 JP 2012178657
(43) Date of publication of application: 12.02.2014
(60) Divisional application: 14163841.1
(73) Proprietor: Toratani Co., Ltd., Kahoku-shi, Ishikawa (JP)
(72) Inventor: Toratani, Ikuo, Kahoku-shi, Ishikawa (JP)
(74) Representative: Fleuchaus, Michael A.

(56) References cited:
- EP-A1- 0 625 316
- US-A- 4 205 397
- US-A1- 2009 049 576
- US-A1- 2012 124 716

## Description

### TECHNICAL FIELD

The present invention relates to clothing such as shorts, pants, a bathing suit, a leotard, briefs, men' s tights, ladies' tights, and paper diapers more specifically, clothing such as shorts which is particularly capable of fitting well to the shape of the bulgy buttocks in the lower region of the hips of a person, preventing hip hemlines thereof from rising when the person is walking or is in a crouching posture or the like, and improving the wearing feeling.

### BACKGROUND ART

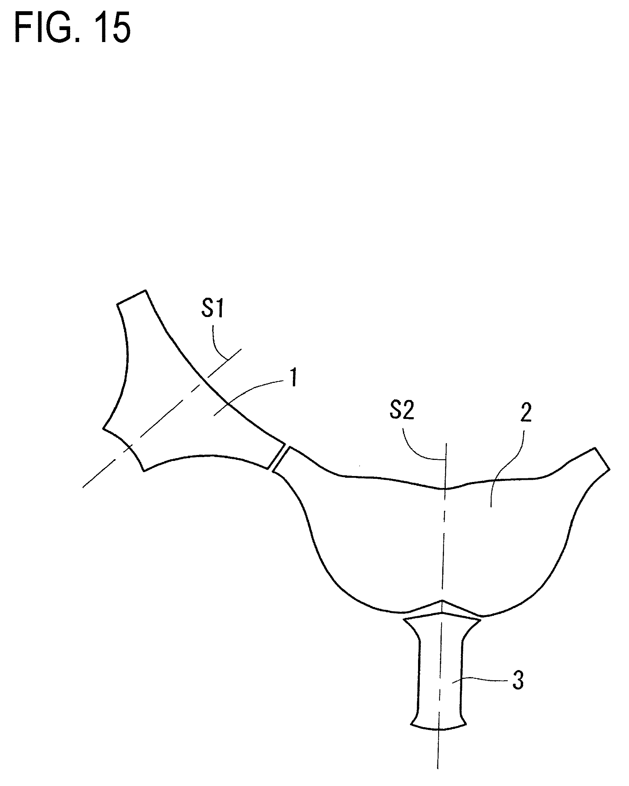

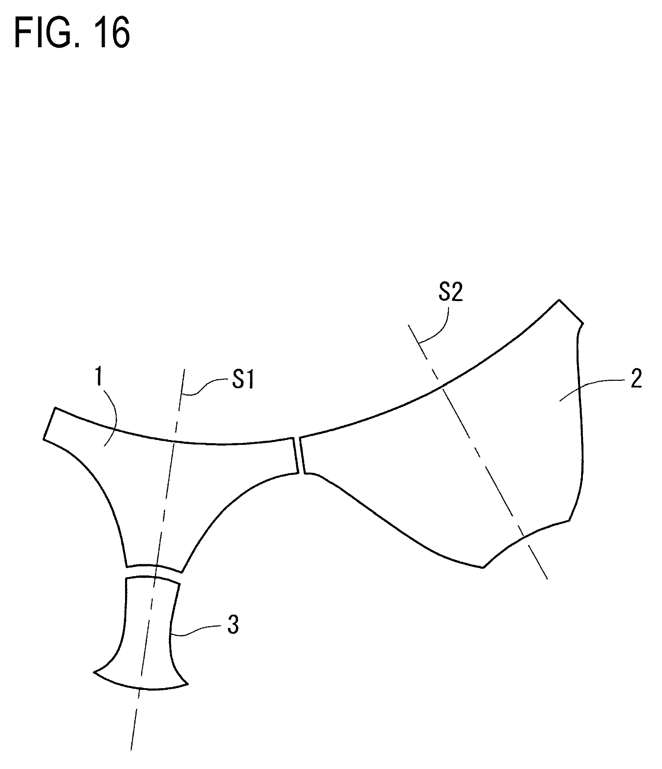

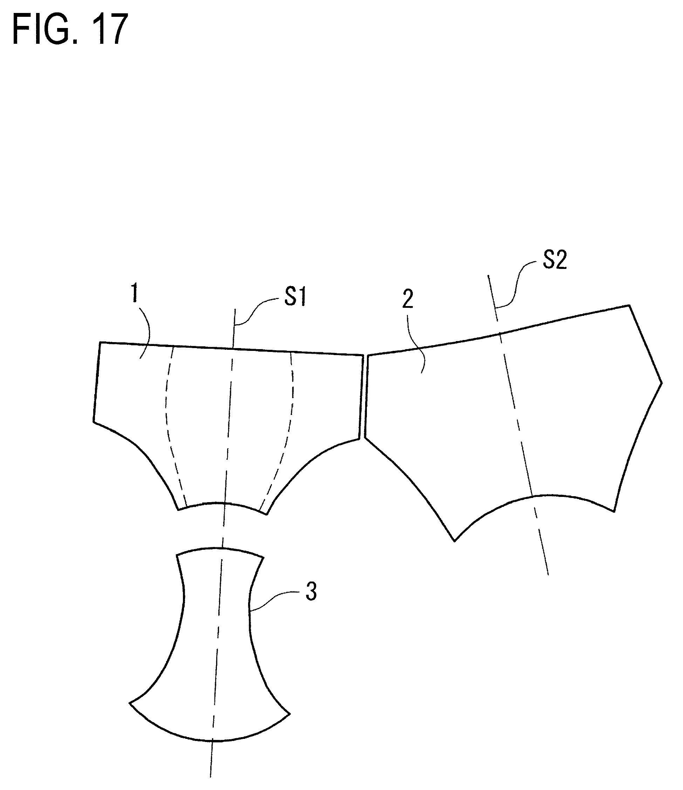

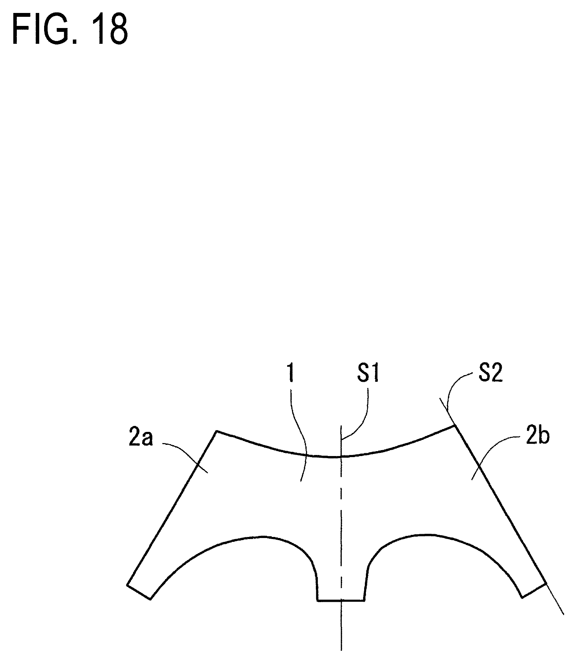

Since the hips are bigger than the waist in the body shape of a person, conventionally, clothing such as shorts of this type is formed by means of patterning (a cutting method) which generally makes the girth of the hips bigger than that of the waist. Specifically, in clothing such as shorts shown in developed form, for example, as shown in FIGS. 15 to 18, with respect to a front center line S1 vertically running through the middle in the lateral direction of a front covering portion (front body) 1, a back center line S2 vertically running through the middle in the lateral direction of a back covering portion (back body) 2 is set so that the downward extended line of the back center line S2 goes away gradually from the downward extended line of the front center line S1 in a downward spreading manner (e.g., refer to Patent Documents 1 to 4). In each of FIGS. 15 to 18, reference numeral 3 denotes a gusset portion disposed between an inner-crotch front portion at the lower end of the front covering portion 1 and an inner-crotch back portion at the lower end of the back covering portion 2. FIG. 15 is a development plan view of a front body 1, a back body 2 and a gusset portion 3 of panties of Patent Document 1, FIG. 16 is a development plan view of a front body 1, a back body 2 and a gusset portion 3 of shorts of Patent Document 2, FIG. 17 is a development plan view of a front body 1, a back body 2 and a gusset portion 3 of shorts of Patent Document 3 and FIG. 18 is a development plan view of a body in which a front body 1 is united at the left and right edges thereof with a left half 2a and a right half 2b of a back body respectively of shorts of Patent Document 4.

Patent Document 1 is Japanese Patent Laid-Open Publication No. 57-205501 (FIG. 1), Patent Document 2 is Japanese Patent Laid-Open Publication No. 7-207502 (FIG. 3), Patent Document 3 is Japanese Patent Laid-Open Publication No. 2000-325396 (FIG. 1) and Patent Document 4 is Japanese Patent Laid-Open Publication No. 11-347067 (FIG. 3).

Further examples of such garments are known from EP 0 625 316 and US 2012/0124716 2

It is certain that the buttock top parts are large in the shape of the hips, and hence, up to the buttock top portion of clothing such as shorts corresponding to the buttock top part, as described above, the back center line S2 of the back covering portion (back body) 2 can also be set to spread downward with respect to the front center line S1 of the front covering portion (front body) 1. However, in the shape of the hips, the lower region of the hips (the shape of the bulgy buttocks in the lower region of the hips) approximates to the shape of the lower half of a sphere, and hence, when the back center line S2 of the back covering portion (back body) 2 is downward spread with respect to the front center line S1 of the front covering portion (front body) 1, the corresponding hip lower portion of clothing such as shorts to the hip lower region does not fit well to the hip lower region. Therefore, when a person changes from a standing posture and sits on a chair, bends at the waist or takes a crouching posture or the like, a hip hemline (a substantially rear half of the inner periphery of each of leg openings) is raised, and when the person is in a standing posture, loose wrinkles or bagginess is easily produced in the portion of clothing such as shorts corresponding to the hip lower region or the gluteal groove part.

### DISCLOSURE OF INVENTION

The invention has been conducted in order to solve the problems. It is an object of the invention to provide clothing such as shorts which is capable of fitting excellently to the lower region of the hips of a person and certainly preventing hip hemlines thereof from rising.

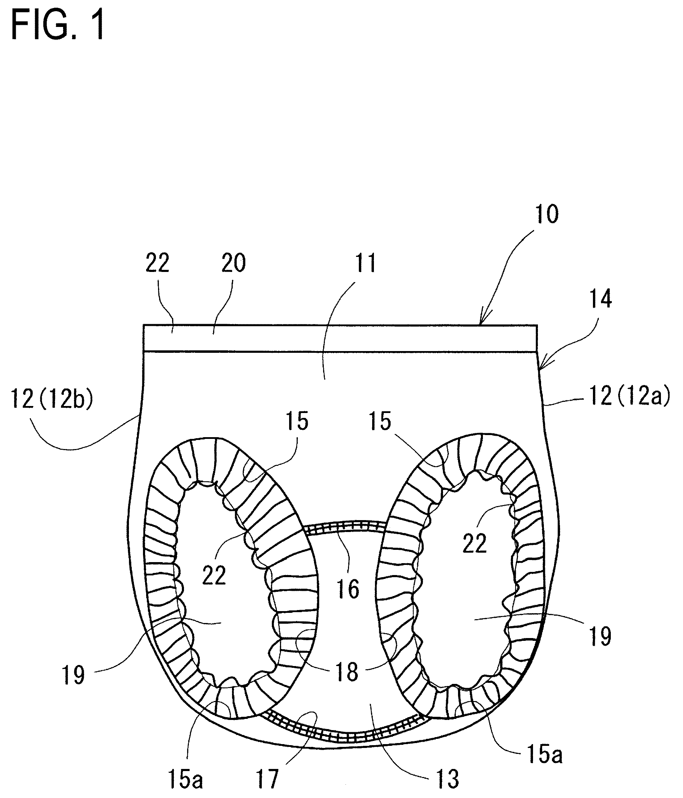

The clothing such as shorts of the invention will be described, with reference to the characters and numerals given to FIGS. 1 to 3 for the purpose of making the contents of the invention easily understandable.

The clothing such as shorts is characterized by including: a body 14 including a stretchable front covering portion 11 covering the lower abdomen of a person and a stretchable back covering portion 12 covering the buttocks of the person, the front covering portion 11 being formed at the lower end thereof with high-thigh-size formation portions 15 which are bilaterally symmetrical and each coved upward; and a gusset portion 13 disposed between a lower end 16 of the front covering portion 11 and a lower end 17 of the back covering portion 12 and covering the crotch of the person, and in that: the body 14 has the left half and the right half of the back covering portion 12 on both left and right sides respectively of the front covering portion 11 and is formed into a tubular shape by joining the left edge of the left half of the back covering portion 12 to the right edge of the right half of the back covering portion 12; and the body 14 is opened and developed along a back center line S2 vertically running through the middle in the lateral direction of the back covering portion 12, and in the developed body 14, with respect to a front center line S1 vertically running through the middle in the lateral direction of the front covering portion 11, the back center line S2 of the back covering portion 12 is set so that the downward extended line of the back center line S2 approaches the downward extended line of the front center line S1 in a downward narrowing manner.

According to the above configurations, the tubular body 14 is opened and developed along the back center line S2 vertically running through the middle in the lateral direction of the back covering portion 12, and in the developed body 14, with respect to the front center line S1 of the front covering portion 11, the back center line S2 of the back covering portion 12 is set so that the downward extended line of the back center line S2 approaches the downward extended line of the front center line S1 in a downward narrowing manner. Therefore, the girth of the portion of the clothing such as shorts corresponding to the hip lower region is narrowed down so that the hip lower portion can fit to the shape of the hip lower region. Hence, the hip lower portion fits to the hemispherical lower half in the hip lower region, or more specifically, the lower portion of the back covering portion 12 fits perfectly to the shape of the bulgy buttocks in the hip lower region so as to envelop the lower half deeply in a squeezing manner. Furthermore, when the person is in a standing posture, neither loose wrinkles nor bagginess is produced in the portion of clothing such as shorts corresponding to the hip lower region or the gluteal groove part, thereby obtaining beautiful hip hemlines. In addition, as is obvious from the description of the configuration, in forming the body 14 into a tubular shape, the number of joining places, the position of joining, the shape of a joining line and the like can be freely chosen without restriction, and further, joining can be even dispensable. This enhances the degree of freedom in design for the appearance of the body (14).

The clothing such as shorts may be configured so that the gusset portion 13 is disposed to be united with the body 14.

The clothing such as shorts may be configured so that if the distance between an intersection point J where the back center line S2 intersects with the lower end 17 of the back covering portion 12 and the front center line S1 is B and the distance between the lower end 16 of the front covering portion 11 and the lower end 17 of the back covering portion 12 is C, then a ratio C/B is set to fall within a range of 0.4 to 2.5. If the ratio C/B is below 0.4, the area where the clothing envelops the buttocks is too small, while if the ratio C/B is above 2.5, the area where it envelops the buttocks is too large. This makes it impossible to set the size around the thigh portion of the clothing to desired measurements, thereby impairing the effects. Further, as a more desired range, the ratio C/B may be set to fall within a range of 0.8 to 1.5.

The clothing such as shorts may be configured so that if the distance between the part nearest to the front center line S1 of a hip hemline 15a which occupies a substantially rear half of the inner periphery of the high-thigh-size formation portion 15 and the front center line S1 is A and the distance between an intersection point J where the back center line S2 intersects with the lower end 17 of the back covering portion 12 and the front center line S1 is B, then a ratio B/A is set to fall within a range of 1.6 to 6. If the ratio B/A is below 1.6, the area where the clothing envelops the buttocks is too small to envelop the buttocks stably. On the other hand, if the ratio B/A is above 6, then the clothing loosely narrows downward, and thereby, a three-dimensional shape is difficult to obtain. Further, as a more desired range, as set forth in a ninth aspect of the invention, the ratio B/A may be set to fall within a range of 2 to 4.

According to the invention, the girth of the portion of the clothing such as shorts corresponding to the hip lower region is narrowed down so that the hip lower portion can fit to the shape of the hip lower region. Therefore, the clothing such as shorts has advantages in that it is capable of deeply and three-dimensionally enveloping the hemispherical lower half in the hip lower region, fitting excellently to the hip lower region and thereby certainly preventing the hip hemlines from rising, and when the person is in a standing posture, producing neither loose wrinkles nor bagginess in the portion of the clothing such as shorts corresponding to the hip lower region or the gluteal groove part and thereby obtaining beautiful hip hemlines. Moreover, in forming the body into a tubular shape, there are no restrictions on the number of joining places, the position of joining, the shape of a joining line and the like, thereby enhancing the degree of freedom in design for the appearance of the body 14.

### BRIEF DESCRIPTION OF DRAWINGS

FIG. 1 is a front view of shorts of a first embodiment of the invention.
FIG. 2 is a rear view of the shorts.
FIG. 3 is a development plan view of a body and a gusset portion of the shorts.
FIG. 4 is a development plan view of a body and a gusset portion, opened along a back center line S2 of a back covering portion 12, of pants of a second embodiment of the invention.
FIG. 5 is a front view of the pants of FIG. 4.
FIG. 6 is a rear perspective view of the pants of FIG. 4.
FIG. 7 is a front view of shorts of a third embodiment of the invention.
FIG. 8 is a rear view of the shorts.
FIG. 9 is a development plan view of a body and a gusset portion of the shorts.
FIG. 10 is a development plan view of a body and a gusset portion, opened along a back center line S2 of a back covering portion 12, of pants of a fourth embodiment of the invention.
FIG. 11 is a front view of the pants of FIG. 10.
FIG. 12 is a rear perspective view of the pants of FIG. 10.
FIG. 13 is a front perspective view of the pants of a fifth embodiment of the invention.
FIG. 14 is a development plan view of a body and a gusset portion, opened along a back center line S2 of a back covering portion 12, of a sixth embodiment of the invention.
FIG. 15 is a development plan view of a front body, a back body and a gusset portion of panties of a conventional art example.
FIG. 16 is a development plan view of a front body, a back body and a gusset portion of shorts of another conventional art example.
FIG. 17 is a development plan view of a front body, a back body and a gusset portion of shorts of still another conventional art example.
FIG. 18 is a development plan view of a body in which a front body is united with a back body of shorts of still another conventional art example.

### BEST MODE FOR CARRYING OUT THE INVENTION

Preferred embodiments of the invention will be described with reference to the drawings.

In FIGS. 1 to 3, shorts 10 of a first embodiment of the clothing of the invention includes, as main configuring members thereof: a body 14 which has a front covering portion 11 covering the lower abdomen of a person and a back covering portion 12 united with the front covering portion 11 and covering the buttocks of the person; and a gusset portion 13 disposed between a lower end 16 of the front covering portion 11 and a lower end 17 of the back covering portion 12 and covering the crotch of the person. The front covering portion 11 and the back covering portion 12 of the body 14 and the gusset portion 13 are made of stretchable fabric.

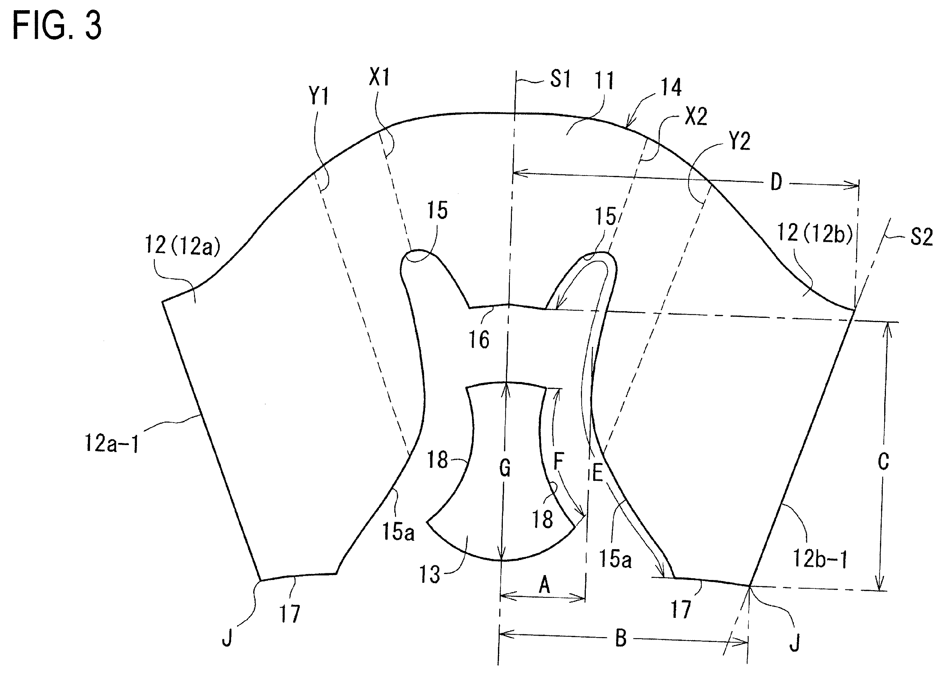

As shown in FIG. 3, the front covering portion 11 is formed at the lower end 16 thereof with high-thigh-size formation portions 15 which are bilaterally symmetrical and each coved upward. A hip hemline 15a which occupies a substantially rear half of the inner periphery of the high-thigh-size formation portion 15 is formed into a convex arcuately curved edge which protrudes toward a front center line S1 vertically running through the middle in the lateral direction of the front covering portion 11.

The front covering portion 11 is united on both left and right sides thereof with a left half 12a and a right half 12b of the back covering portion 12, respectively.

The body 14 is opened and developed along a back center line S2 vertically running through the middle in the lateral direction of the back covering portion 12, and in the developed body 14, with respect to the front center line S1 vertically running through the middle in the lateral direction of the front covering portion 11, the back center line S2 of the back covering portion 12 is set so that the downward extended line of the back center line S2 approaches the downward extended line of the front center line S1 in a downward narrowing manner. As indicated by solid lines in FIG. 3, a left edge 12a-1 of the left half 12a of the back covering portion 12 and a right edge 12b-1 of the right half 12b of the back covering portion 12 are linearly formed in a downward narrowing manner and coincide with the back center line S2 of the back covering portion 12. However, the left edge 12a-1 and the right edge 12b-1 are not limited to the linear shape. Instead of this shape, for example, a substantially lower half of the left edge 12a-1 and a substantially lower half of the right edge 12b-1 may be each formed into a gentle arcuately curved edge which is outward convex in a downward narrowing manner, so as to extend along the curved shape of the hip lower region as far as possible.

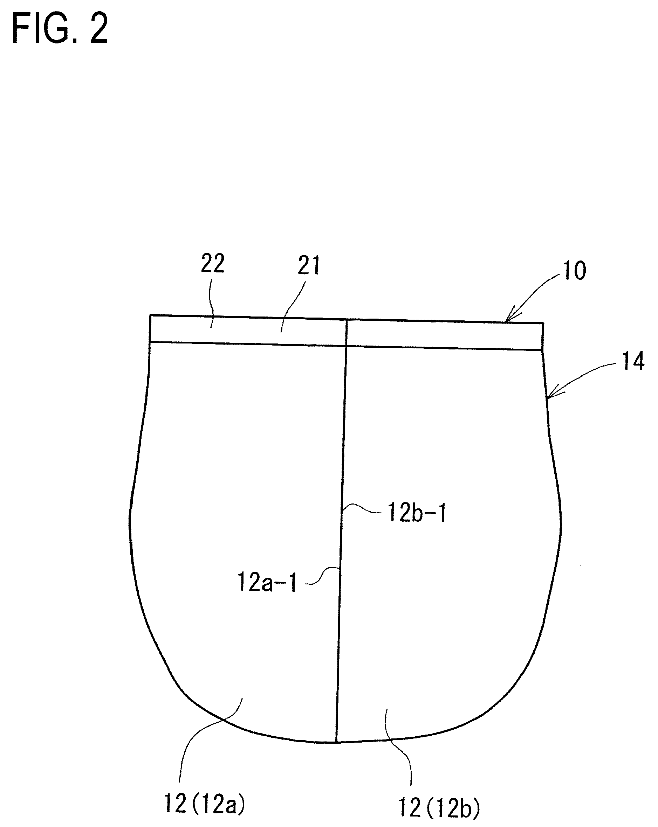

The body 14 cut out in this way is formed, as shown in FIG. 2, into a tubular shape by joining the left edge 12a-1 of the left half 12a of the back covering portion 12 to the right edge 12b-1 of the right half 12b of the back covering portion 12. The lower end 16 of the front covering portion 11 and the lower end 17 of each of the left half 12a and the right half 12b of the back covering portion 12 are connected to each other by the gusset portion 13. Consequently, as shown in FIG. 1, right and left leg openings 19 are formed by the right and left high-thigh-size formation portions 15 and right and left edges 18 of the gusset portion 13, respectively.

A stretchable strip-like material 22 such as a stretch lace, a fabric and a rubber, which is slightly wide (if it is a narrow type, the effect is reduced), is joined to each of waist portions 20, 21 of the front covering portion 11 and the back covering portion 12 of the body 14 respectively and the peripheries of the leg openings 19.

In the thus joined shorts 10, the body 14 is opened and developed along the back center line S2 of the back covering portion 12, and in the developed body 14, with respect to the front center line S1 of the front covering portion 11, the back center line S2 of the back covering portion 12 is set so that the downward extended line of the back center line S2 approaches the downward extended line of the front center line S1 in a downward narrowing manner. Therefore, the body 14 is formed into a tubular shape so that the vicinity of the hip hem can be three-dimensionally narrowed down in a non-worn state. As a result, in a worn state, the body 14 fits to the shape of the hip lower region and the hip hemlines 15a fit to the gluteal groove part of the hips, so that the body 14 can envelop the hip lower region deeply and sufficiently. Accordingly, when the person changes from a standing posture and sits on a chair, bends at the waist or takes a crouching posture or the like, the hip hemlines 15a can be surely prevented from rising. In addition, when the person is in a standing posture, neither loose wrinkles nor bagginess is produced in the portion of the clothing such as shorts corresponding to the hip lower region or the gluteal groove part, thereby obtaining beautiful hip hemlines.

In the above developed form, with respect to the front center line S1 of the front covering portion 11, the back center line S2 of the back covering portion 12 is set so that the downward extended line of the back center line S2 approaches the downward extended line of the front center line S1 in a downward narrowing manner, and hence, the measurements of the buttock portion and around the thigh portion of the shorts 10 are secured in biased direction. However, the front covering portion 11, the back covering portion 12 and the gusset portion 13 are made of stretchable fabric, thereby enabling the person to wear the shorts 10 without any trouble.

In FIG. 3, if a distance A (the distance between the part nearest to the front center line S1 of the hip hemline 15a and the front center line S1; the same below) is shortened and a distance C (the distance between the lower end 16 and the lower end 17; the same below) is lengthened, then the effect of enveloping the buttocks from aside can be obtained. A distance B (the distance between an intersection point J where the back center line S2 intersects with the lower end 17 and the front center line S1; the same below) may be preferably lengthened to secure volume enough to envelop the buttocks. However, if the distance B is too long, then the back center lines S2 less narrow downward with respect to the front center line S1, and thereby, a three-dimensional shape is difficult to obtain.

Therefore, a predetermined high-thigh size is decided, and next, it is necessary to obtain an ideal pattern in consideration of the above distances A, B, C.

Specifically, if the ratio B/A is low, the area where the shorts 10 envelop the buttocks is too small to envelop the buttocks stably. On the other hand, if the ratio B/A is too high, then the shorts 10 loosely narrow downward, and thereby, a three-dimensional shape is difficult to obtain. Taking this into account, the ratio B/A may be set to fall within a range of 1.6 to 6, more preferably 2 to 4.

Further, if the ratio C/B is too low, then in the same manner as the case where the ratio B/A is low, the area where the shorts 10 envelop the buttocks is too small. On the other hand, if the ratio C/B is too high, the area where they envelop the buttocks is too large. This makes it impossible to set the size around the thigh portion of the shorts 10 to desired measurements, thereby impairing the effects. Taking this into account, the ratio C/B may be set to fall within a range of 0.4 to 2.5, more preferably 0.8 to 1.5.

Concretely, as an example of the measurements, the distance A is dimensioned to 7 cm, the distance B is dimensioned to 18 cm, the distance C is dimensioned to 20 cm, a distance D (the distance between the uppermost end of the left edge 12a-1 of the back covering portion 12 and the front center line S1 of the front covering portion 11, the distance between the uppermost end of the right edge 12b-1 of the back covering portion 12 and the front center line S1 of the front covering portion 11; the same below) is dimensioned to 25 cm, a length E (the full length of the high-thigh-size formation portion 15) is dimensioned to 33 cm, a length F (the length of the left edge 18, the length of the right edge 18 of the gusset portion 13) is dimensioned to 12 cm and a length G (the vertical length of the middle of the gusset portion 13) is dimensioned to 14 cm.

In the embodiment, the body 14 shown in FIG. 3 has the left half and the right half of the back covering portion 12 united with the front covering portion 11 on both left and right sides thereof respectively and is formed into a tubular shape by joining the left edge of the left half of the back covering portion 12 to the right edge of the right half of the back covering portion 12. However, the invention is not limited to this and may be configured as follows.

In addition to the place of the back center line S2 of the back covering portion 12, the body 14 may be joined to the place of the front center line S1 of the front covering portion 11, or alternatively, as indicated by the broken lines of FIG. 3, to the places on both left and right sides X1, X2, or Y1, Y2 of the front covering portion 11 or another suitable place.

In sum, the body 14 may be joined to at least one place S1, X1, X2, Y1, Y2 in the peripheral direction thereof, in addition to the place of the back center line S2 of the back covering portion 12, and the body 14 is opened and developed along the back center line S2 of the back covering portion 12 and in the developed body 14, the back center line S2 of the back covering portion 12 may be set with respect to the front center line S1 of the front covering portion 11 so that the downward extended line of the back center line S2 approaches the downward extended line of the front center line S1 in a downward narrowing manner.

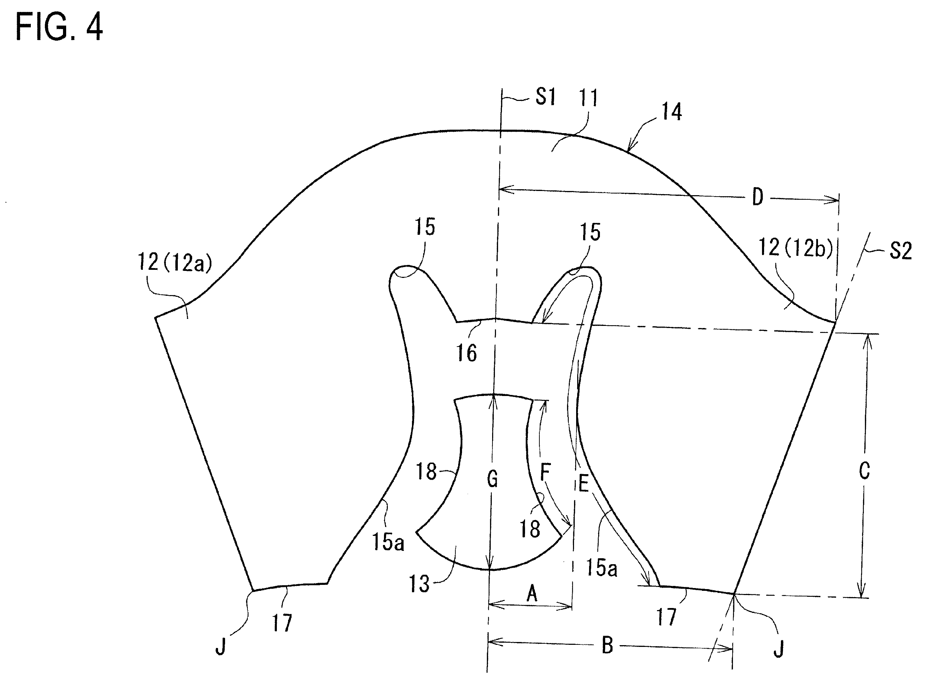

Furthermore, the clothing as the target of the invention is not limited to the clothing including the body 14 shown in FIG. 3 and may be the clothing including the body 14 shown in FIG. 4.

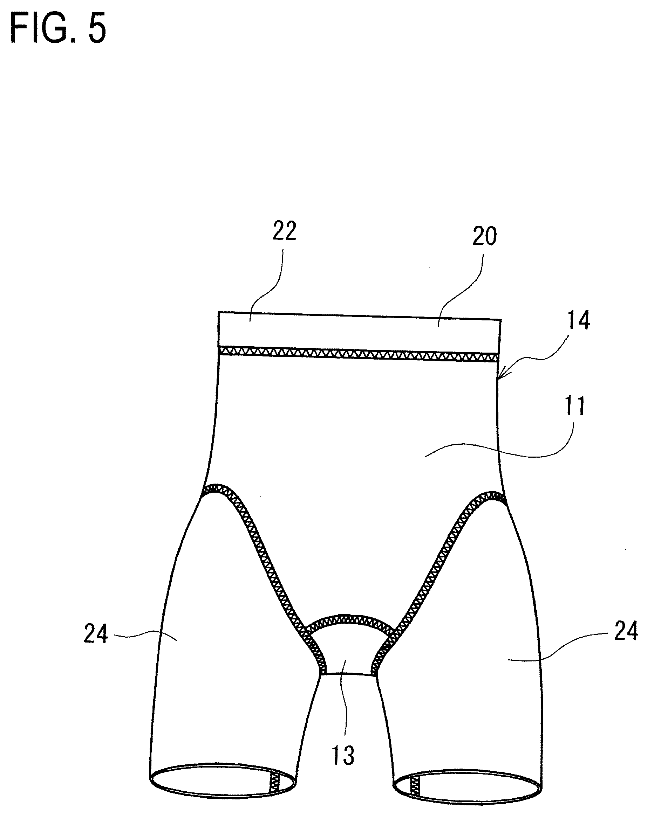

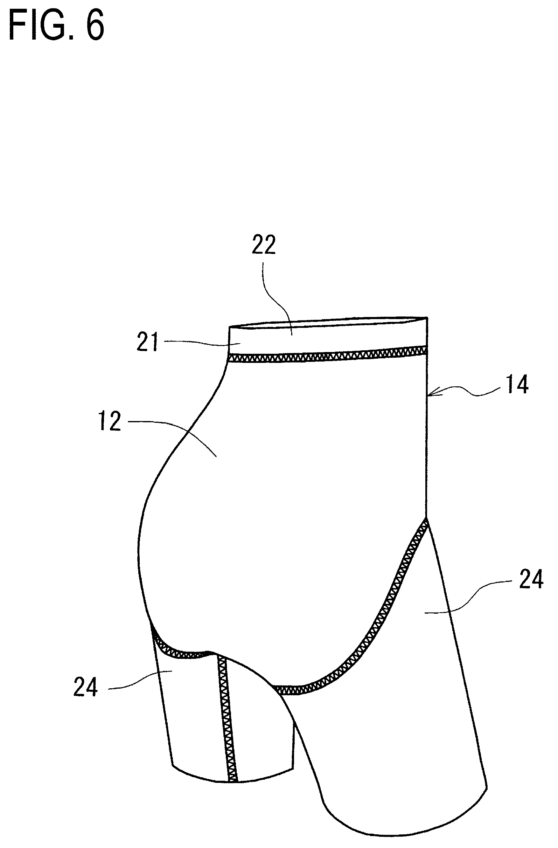

Specifically, the body 14 of FIG. 4 has the back covering portion 12 united with the front covering portion 11 on both left and right sides thereof, and hence, as shown in FIGS. 5 and 6, it may be a body formed into a tubular shape without a joining place.

Even this body 14 has the same operation and effects as the shorts of FIGS. 1 to 3, as shown in FIG. 4, as long as the body 14 is cut, opened and developed along the back center line S2 of the back covering portion 12 and in the developed body 14, the back center line S2 of the back covering portion 12 is set with respect to the front center line S1 of the front covering portion 11 so that the downward extended line of the back center line S2 approaches the downward extended line of the front center line S1 in a downward narrowing manner (the pants shown in FIGS. 5 and 6 are configured so that each of thigh covering portions 24 can be joined thereto).

Moreover, in the clothing as the target of the invention, the gusset portion 13 may be united with the body 14.

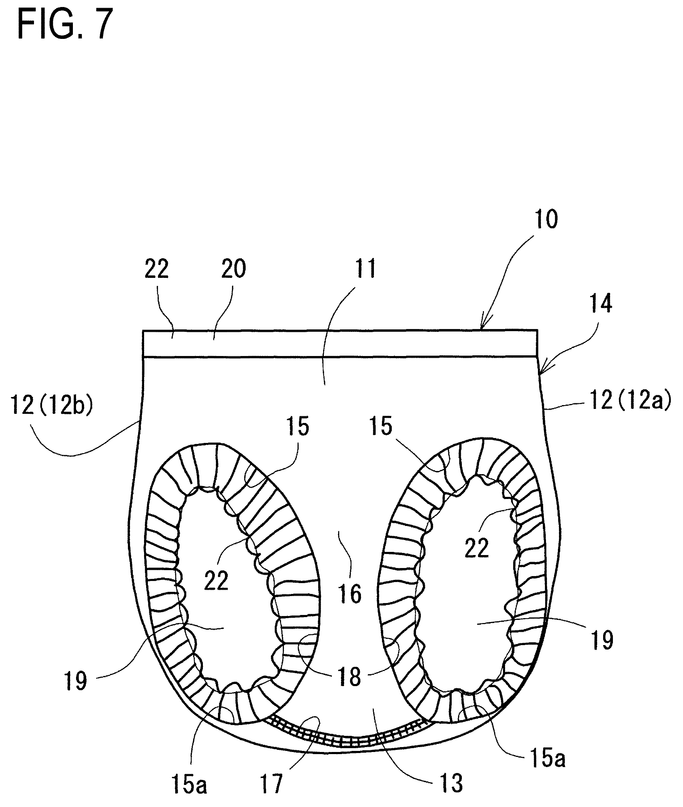

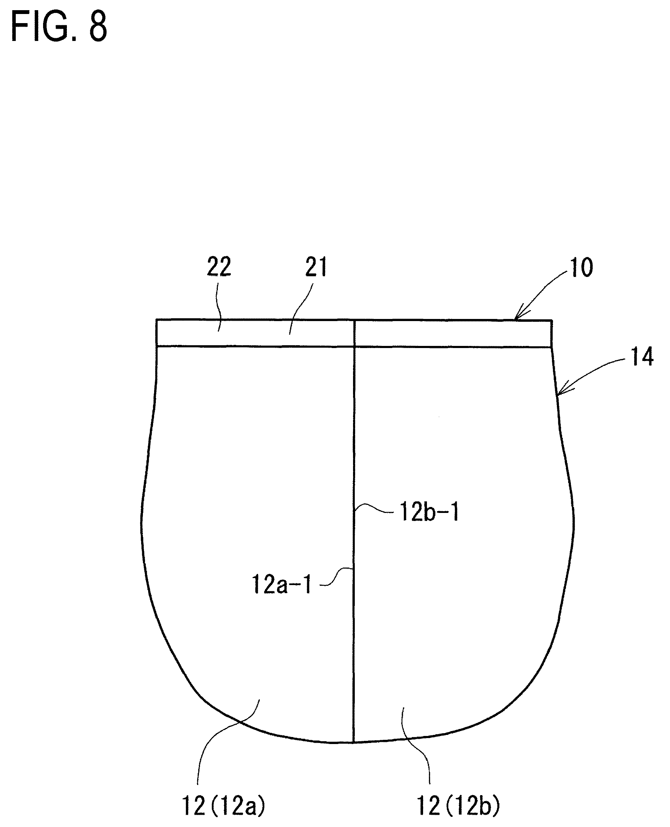

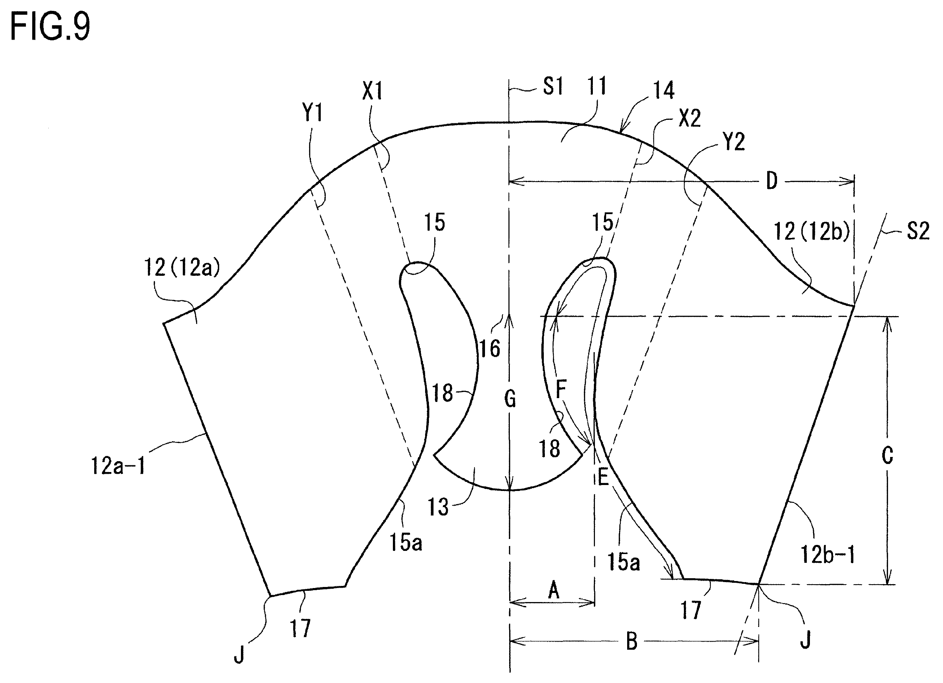

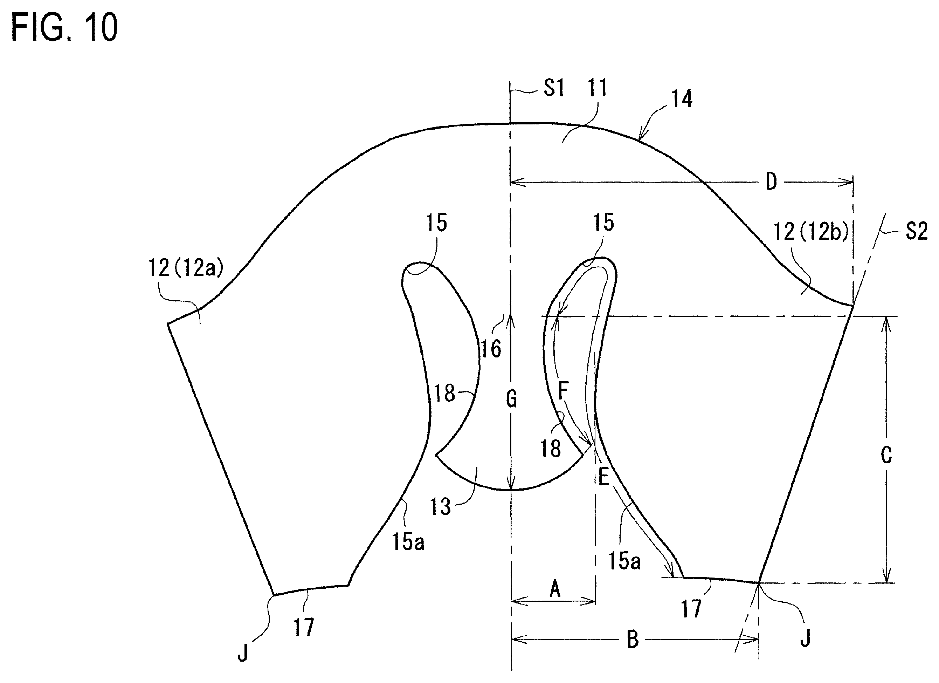

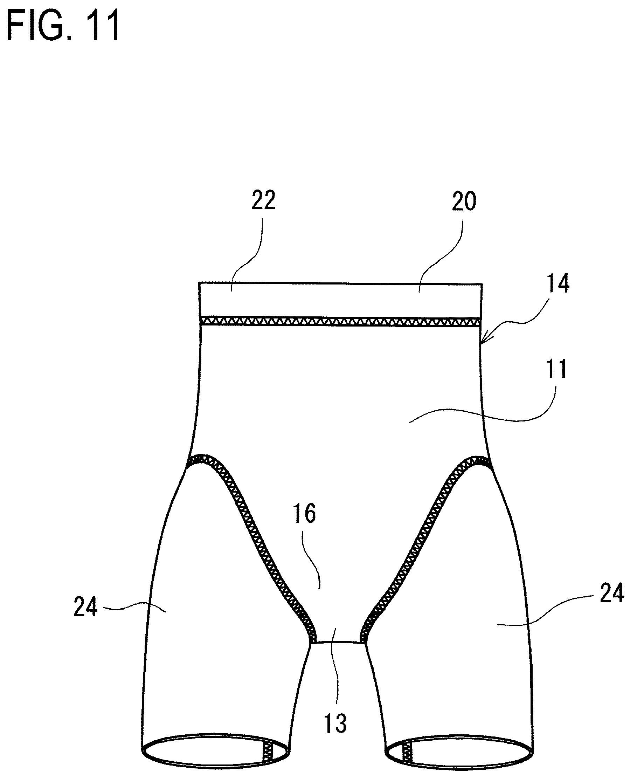

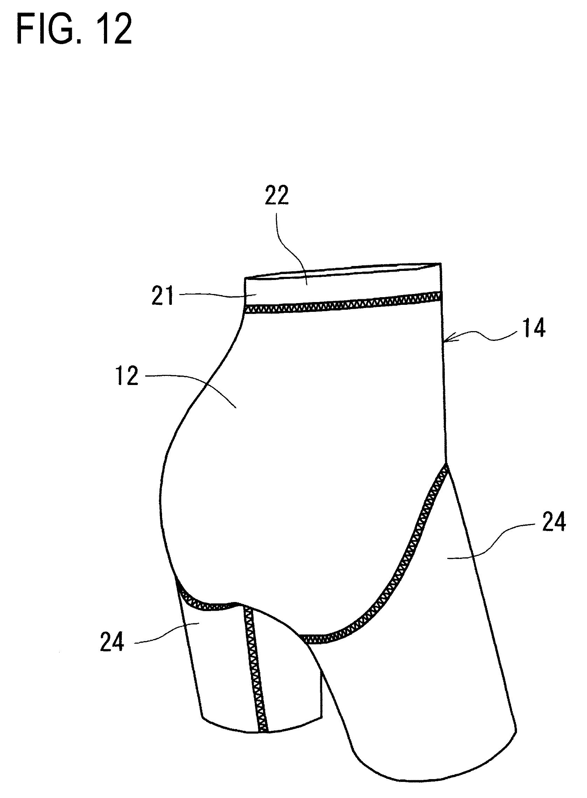

For example, as shown in FIGS. 7, 8, 9, 10, 11, and 12, the gusset portion 13 may be configured as a gusset portion united with a body, which is continuous and united with the lower end 16 of the front covering portion 11. In view of configuration except one of the gusset portion 13, the clothing of the third embodiment shown in FIGS. 7, 8 and 9 is the substantially same as the clothing of the first embodiment shown in FIGS. 1, 2, and 3. The clothing of the fourth embodiment shown in FIGS. 10, 11 and 12 is the substantially same as the clothing of the second embodiment shown in FIGS. 4, 5, and 6.

Moreover, in case that the gusset portion 13 is united with the body 14, the gusset portion 13 may be configured in a form of gusset portion united with a body, which is joined to the lower end 16 of the front covering portion 11 and is continuous and united with the lower end 17 of the back covering portion 12. Alternatively, the gusset portion 13 may be configured in still another form of a gusset portion united with a body, which is continuous with both of the lower end 16 of the front covering portion 11 and the lower end 17 of the back covering portion 12.

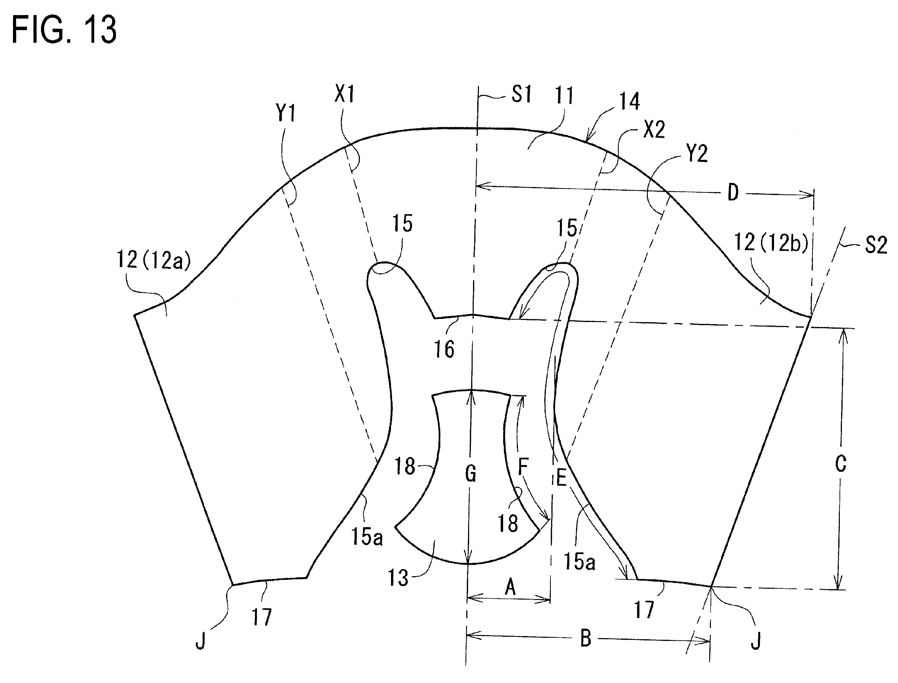

In addition, the clothing as the target of the invention is not limited to the clothing including the body 14 separated from the gusset portion shown in FIG. 3 and may be the clothing including the body 14 separated from the gusset portion shown in FIG. 13.

Specifically, the body 14 may be a body which is united at the place of the back center line S2 of the back covering portion 12 and which is joined, except for the back center line S2, to the place of the front center line S1, or alternatively, as indicated by the broken lines of FIG. 13, to the places on both left and right sides X1, X2, or Y1, Y2 of the front covering portion 11 or another suitable place.

In sum, the body 14 may be joined to at least one place in the peripheral direction thereof which is other than the place of the back center line S2 of the back covering portion 12, and the body 14 is opened and developed along the back center line S2 of the back covering portion 12 and in the developed body 14, the back center line S2 of the back covering portion 12 may be set with respect to the front center line S1 of the front covering portion 11 so that the downward extended line of the back center line S2 approaches the downward extended line of the front center line S1 in a downward narrowing manner.

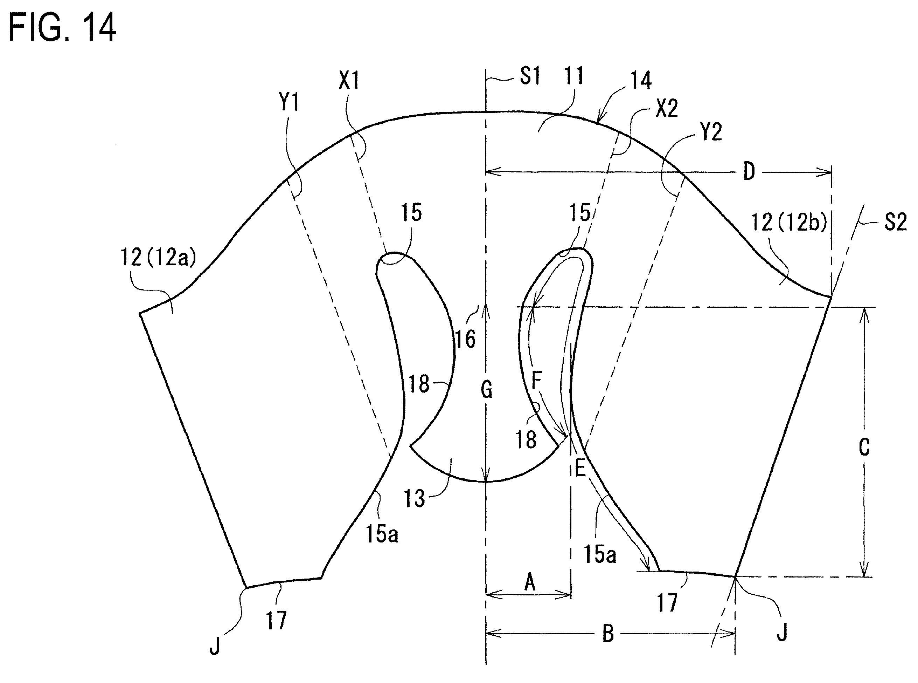

If the gusset portion 13 is united with a body, which is disposed to be continuous with both of the lower end 16 of the front covering portion 11 and the lower end 17 of the back covering portion 12, it is preferable and easier to manufacture the product of the invention that the body 14 is united with both of a place of the front center line S1 of the front covering portion 11 and a place of the back center line S2 of the back covering portion 12 so as not to be joined, as shown in FIG. 14. As indicated by the broken lines in FIG. 14, it is preferable that the body 14 may be joined at least to the places on both left and right sides X1, X2 of the front covering portion 11 or both left and right sides Y1, Y2 of the back covering portion 12.

In sum, also in this case, the body 14 may be joined to at least one place in the peripheral direction thereof, except for the back center line S2 of the back covering portion 12, and the body 14 is cut, opened and developed along the back center line S2 of the back covering portion 12 and in the developed body 14, the back center line S2 of the back covering portion 12 may be set with respect to the front center line S1 of the front covering portion 11 so that the downward extended line of the back center line S2 approaches the downward extended line of the front center line S1 in a downward narrowing manner.

Furthermore, in terms of the body 14 of the clothing as the target of the invention, in order to prevent bagginess in the body 14 and neaten the appearance and design thereof on the sides of the waist portions 20, 21 shown in FIGS. 1, 2, 5, 6, 7, 8, 11 and 12, the body 14 is opened and developed along the back center line S2 of the back covering portion 12 and in the developed form, the body 14 may be shaped so that the portion thereof on the upper side of the back center line S2 contracts toward the front center line S1. Moreover, the body 14 may be shaped so that, among the upper side and the lower side of the back center line S2, the upper side is substantially parallel to the front center line S1.

Moreover, the clothing as the target of the invention may be similarly applied to a bathing suit, a leotard, tights for men or ladies, briefs for men, paper diapers or the like. Still further, the body 14 may also be provided with darts.

## Claims

1. Clothing such as shorts, comprising:
a tubular body (14) including a stretchable front covering portion (11) covering the lower abdomen of a person and a stretchable back covering portion (12) covering the buttocks of the person, the front covering portion (11) being formed at the lower end thereof with high-thigh-size formation portions (15) which are bilaterally symmetrical and each coved upward; and
a gusset portion (13) disposed between a lower end (16) of the front covering portion (11) and a lower end (17) of the back covering portion (12) and covering the crotch of the person,
**characterised in that**
when the body (14) is opened and developed along a back center line (S2) vertically running through the middle in the lateral direction of the back covering portion (12), the developed body (14) has a configuration that, with respect to a front center line (S1) vertically running through the middle in the lateral direction of the front covering portion (11), the back center line (S2) of the back covering portion (12) is set so that the downward extended line of the back center line (S2) approaches the downward extended line of the front center line (S1) in a downward narrowing manner.

2. The clothing such as shorts according to claim 1, wherein the gusset portion (13) is disposed to be united with the body (14).

3. The clothing such as shorts according to claims 1 or 2, wherein the body (14) is opened and developed along the back center line (S2) vertically running through the middle in the lateral direction of the back covering portion (12), and in the developed body (14), if the distance between an intersection point (J) where the back center line (S2) intersects with the lower end (17) of the back covering portion (12) and the front center line (S1) is B and the distance between the lower end (16) of the front covering portion (11) and the lower end (17) of the back covering portion (12) is C, then a ratio C/B is set to fall within a range of 0.4 to 2.5.

4. The clothing such as shorts according to any of claims 1 to 3, wherein the body (14) is opened and developed along the back center line (S2) vertically running through the middle in the lateral direction of the back covering portion (12), and in the developed body (14), if the distance between an intersection point (J) where the back center line (S2) intersects with the lower end (17) of the back covering portion (12) and the front center line (S1) is B and the distance between the lower end (16) of the front covering portion (11) and the lowerend (17) of the back covering portion (12) is C, then a ratio C/B is set to fall within a range of 0.8 to 1.5.

5. The clothing such as shorts according to any of claims 1 to 4, wherein the body (14) is opened and developed along the back center line (S2) vertically running through the middle in the lateral direction of the back covering portion (12), and in the developed body (14), if the distance between the part nearest to the front center line (S1) of a hip hemline (15a) on the inner periphery of the high-thigh-size formation portion (15) and the front center line (S1) is A and the distance between an intersection point (J) where the back center line (S2) intersects with the lower end (17) of the back covering portion (12) and the front center line (S1) is B, then a ratio B/A is set to fall within a range of 1.6 to 6.

6. The clothing such as shorts according to any of claims 1 to 4, wherein the body (14) is opened and developed along the back center line (S2) vertically running through the middle in the lateral direction of the back covering portion (12), and in the developed body (14), if the distance between the part nearest to the front center line (S1) of a hip hemline (15a) on the inner periphery of the high-thigh-size formation portion (15) and the front center line (S1) is A and the distance between an intersection point (J) where the back center line (S2) intersects with the lower end (17) of the back covering portion (12) and the front center line (S1) is B, then a ratio B/A is set to fall within a range of 2 to 4.

## Patentansprüche

1. Bekleidung wie etwa eine kurze Hose, umfassend:
einen röhrenförmigen Hauptteil (14) mit einem dehnbaren Vorderseiten-Bedeckungsabschnitt (11), der den unteren Bauch einer Person bedeckt, und einem dehnbaren Rückseiten-Bedeckungsabschnitt (12), derdas Gesäß der Person bedeckt, wobei der Vorderselten-Bedeckungsabschnitt (11) am unteren Ende desselben mit Oberschenkel-Ausbildungsabschnitten (15) gebildet ist, die bilateral symmetrisch und Jeweils aufwärts gewölbt sind; und
einen Zwickelabschnitt (13), der zwischen einem unteren Ende (16) des Vorderseiten-Bedeckungsabschnitts (11) und einem unteren Ende (17) des Rückseiten-Bedeckungsabschnitts (12) angeordnet ist und den Schritt der Person bedeckt,
**dadurch gekennzeichnet, dass**,
wenn der Hauptteil (14) entlang einer rückseitigen Mittellinie (S2) geöffnet und entfaltet ist, die durch die Mitte in der Querrichtung des Rückseiten-Bedeckungsabschnitts (12) vertikal verläuft, der entfaltete Hauptteil (14) eine Gestaltung hat, bei welcher mit Bezug auf eine vordere Mittellinie (S1), die durch die Mitte in der Querrichtung des Vorderseiten-Bedeckungsabschnitts (11) vertikal verläuft, die rückseitige Mittellinie (S2) des Rückseiten-Bedeckungsabschnitts (12) so eingestellt ist, dass die nach unten verlängerte Linie der rückseitigen Mittellinie (S2) sich der nach unten verlängerten Linie der vorderen Mittellinie (S1) nach unten zulaufend nähert.

2. Bekleidung wie etwa eine kurze Hose gemäß Anspruch 1, bei welcher der Zwickelabschnitt (13) so eingerichtet ist, dass derselbe mit dem Hauptteil (14) eintellig ist.

3. Bekleidung wie etwa eine kurze Hose gemäß Anspruch 1 oder 2, bei welcher der Hauptteil (14) entlang der rückseitigen Mittellinie (S2) geöffnet und entfaltet ist, die durch die Mitte in der Querrichtung des Rückseiten-Bedeckungsabschnitts (12) vertikal verläuft, und in dem entfalteten Hauptteil (14), wenn die Distanz zwischen einem Schnittpunkt (J), an dem die rückseitige Mittellinie (S2) sich mit dem unteren Ende (17) des Rückseiten-Bedeckungsabschnitts (12) schneidet, und der vorderen Mittellinie (S1) B ist und die Distanz zwischen dem unteren Ende (16) des Vorderseiten-Bedeckungsabschnitts (11) und dem unteren Ende (17) des Rückseiten-Bedeckungsabschnitts (12) C ist, ein Verhältnis C/B so eingestellt ist, dass dasselbe In einen Bereich von 0,4 bis 2,5 fällt.

4. Bekleidung wie etwa eine kurze Hose gemäß einem der Ansprüche 1 bis 3, bei welcher der Hauptteil (14) entlang der rückseitigen Mittellinie (S2) geöffnet und entfaltet ist, die durch die Mitte in der Querrichtung des Rückseiten-Bedeckungsabschnitts (12) vertikal verläuft, und in dem entfalteten Hauptteil (14), wenn die Distanz zwischen einem Schnittpunkt (J), an dem die rückseitige Mittellinie (S2) sich mit dem unteren Ende (17) des Rückseiten-Bedeckungsabschnitts (12) schneide, und der vorderen Mittellinie (S1) B ist und die Distanz zwischen dem unteren Ende (16) des Vorderseiten-Bedeckungsabschnitts (11) und dem unteren Ende (17) des Rückseiten-Bedeckungsabschnitts (12) C ist, ein Verhältnis C/B so eingestellt ist, dass dasselbe in einen Bereich von 0,8 bis 1,5 fällt.

5. Bekleidung wie etwa eine kurze Hose gemäß einem der Ansprüche 1 bis 4, bei welcher der Hauptteil (14) entlang der rückseitigen Mittellinie (S2) geöffnet und entfaltet ist, die durch die Mitte in der Querrichtung des Rückseiten-Bedeckungsabschnitts (12) vertikal verläuft, und in dem entfalteten Hauptteil (14), wenn die Distanz zwischen dem am nächsten an der vorderen Mittellinie (S1) gelegenen Teil eines Hüftsaums (15a) auf dem Innenumfang des Oberschenkel-Ausbildungsabschnitts (15) und der vorderen Mittellinie (51) A ist und die Distanz zwischen einem Schnittpunkt (J), an dem die rückseitige Mittellinie (S2) sich mit dem unteren Ende (17) des Rückseiten-Bedeckungsabschnitts (12) schneidet, und der vorderen Mittellinie (S1) B ist, ein Verhältnis B/A so eingestellt ist, dass dasselbe in einen Bereich von 1,6 bis 6 fällt.

6. Bekleidung wie etwa eine kurze Hose gemäß einem der Ansprüche 1 bis 4, bei welcher der Hauptteil (14) entlang der rückseitigen Mittellinie (S2) geöffnet und entfaltet ist, die durch die Mitte In der Querrichtung des Rückseiten-Bedeckungsabschnitts (12) vertikal verläuft, und in dem entfalteten Hauptteil (14), wenn die Distanz zwischen dem am nächsten an der vorderen Mittellinie (S1) gelegenen Teil eines Hüftsaums (15a) auf dem Innenumfang des Oberschenkel-Ausbildungsabschnitts (15) und der vorderen Mittellinie (S1) A ist und die Distanz zwischen einem Schnittpunkt (J), an dem die rückseitige Mittellinie (S2) sich mit dem unteren Ende (17) des Rückseiten-Bedeckungsabschnitts (12) schneidet, und der vorderen Mittellinie (S1) B ist, ein Verhältnis B/A so eingestellt ist, dass dasselbe in einen Bereich von 2 bis4 fällt.

## Revendications

1. Vêtement tel qu'un short, comprenant :
un corps tubulaire (14) comportant une partie de recouvrement avant extensible (11) couvrant l'abdomen inférieur d'une personne et une partie de recouvrement arrière extensible (12) couvrant les fesses de la personne, la partie de recouvrement avant (11) étant formée à son extrémité inférieure avec des parties de formation à haute taille de cuisse (15) qui sont bilatéralement symétriques etchacune à cavets vers le haut ; et
une partie en gousset (13) disposée entre une extrémité inférieure (16) de la partie de recouvrement avant (11) et une extrémité inférieure (17) de la partie de recouvrement arrière (12) et couvrant l'entrejambe de la personne,
**caractérisé en ce que**,
lorsque le corps (14) est ouvert et développé le long d'une ligne centrale arrière (S2) circulant verticalement à travers le centre dans la direction latérale de la partie de recouvrement arrière (12), le corps développé (14) possède une configuration selon laquelle, par rapport à une ligne centrale avant (S1) circulant verticalement à travers le centre dans la direction latérale de la partie de recouvrement avant (11), la ligne centrale arrière (S2) de la partie de recouvrement arrière (12) est déterminée afin que la ligne s'étendant vers le bas de la ligne centrale arrière (S2) se rapproche de la ligne s'étendant vers le bas de la ligne centrale avant (S1) d'une manière se rétrécissant vers le bas.

2. Vêtement tel qu'un short selon la revendication 1, dans lequel la partie de gousset (13) est disposée afin d'être unie avec le corps (14).

3. Vêtement tel qu'un short selon les revendications 1 ou 2, dans lequel le corps (14) est ouvert et développé le long de la ligne centrale arrière (S2) circulant verticalement à travers le centre dans la direction latérale de la partie de recouvrement arrière (12), et dans le corps développé (14), si la distance entre un point d'intersection (J) où la ligne centrale arrière (S2) fait intersection avec l'extrémité inférieure (17) de la partie de recouvrement arrière (12) et la ligne centrale avant (S1) est B et la distance entre l'extrémité inférieure (16) de la partie de recouvrement avant (11) et l'extrémité inférieure (17) de la partie de recouvrement arrière (12) est C, alors un rapport C/B est déterminé afin de tomber dans une gamme de 0,4 2,5.

4. Vêtement tel qu'un short selon une quelconque des revendications 1 3, dans lequelle corps (14) est ouvert et développé le long de la ligne centrale arrière (52) circulant verticalement à travers le centre dans la direction latérale de la partie de recouvrement arrière (12), et dans le corps développé (14), si la distance entre un point d'intersection (J) où la ligne centrale arrière (S2) fait intersection avec l'extrémité inférieure (17) de la partie de recouvrement arrière (12) et la ligne centrale avant (S1) est B et la distance entre l'extrémité inférieure (16) de la partie de recouvrement avant (11) et l'extrémité inférieure (17) de la partie de recouvrement arrière (12) est C, alors un rapport C/B est déterminé afin de tomber dans une gamme de 0,8 à 1,5.

5. Vêtement tel qu'un short selon une quelconque des revendications 1 à 4, dans lequel le corps (14) est ouvert et développé le long de la ligne centrale arrière (S2) circulant verticalement à travers le centre dans la direction latérale de la partie de recouvrement arrière (12), et dans le corps développé (14), si la distance entre la partie la plus proche de la ligne centrale avant (S1) d'un ourlet de hanche (15a) sur la périphérie interne de la partie de formation à haute taille de cuisse (15) et la ligne centrale avant (S1) est A et la distance entre un point d'intersection (J) où la ligne centrale arrière (S2) fait intersection avec l'extrémité Inférieure (17) de la partie de recouvrement arrière (12) et la ligne centrale avant (S1) est B, alors un rapport B/A est déterminé afin de tomber dans une gamme de 1,6 à 6.

6. Vêtement tel qu'un short selon une quelconque des revendications 1 à 4, dans lequel le corps (14) est ouvert et développé le long de la ligne centrale arrière (S2) circulant verticalement à travers le centre dans la direction latérale de la partie de recouvrement arrière (12), et dans le corps développé (14), si la distance entre la partie la plus proche de la ligne centrale avant (S1) d'un ourlet de hanche (15a) sur la périphérie interne de la partie de formation à haute taille de cuisse (15) et la ligne centrale avant (S1) est A et la distance entre un point d'intersection (J) où la ligne centrale arrière (S2) fait Intersection avec l'extrémité inférieure (17) de la partie de recouvrement arrière (12) et la ligne centrale avant (S1) est B, alors un rapport B/A est déterminé afin de tomber dans une gamme de 2 à 4,
